# EUROPEAN PATENT APPLICATION

(11) **EP 4 494 559 A1**
(43) Date of publication of application: **22.01.2025**
(21) Application number: 23186230.1
(22) Date of filing: 18.07.2023
(51) Int. Cl.: A61B 5/349, G16H 50/20, G16H 50/30, A61B 5/00

(54) **COMPUTER-IMPLEMENTED METHOD FOR THE ESTIMATION OF A RISK OF HEART RHYTHM DISORDER IN A PATIENT'S HEART**

(71) Applicant: Inheart, 33600 Pessac (FR)
(72) Inventor: PEYRAT, Jean-Marc, 33600 PESSAC (FR)
(74) Representative: Aquinov

(57) **Abstract**

The invention concerns a computer-implemented method for the estimation of a risk of heart rhythm disorder in a patient's heart, the method comprising: step of receiving at least one mapping of points representing said tissue of said patient's heart, each point being labelled with at least a value of at least one parameter and/or a classification indicating a local characteristic of said tissue at or around said point position; step of receiving a set of electrograms signals, said set of electrograms signals being associated to a spatial location of said patient's heart; step of registering said spatial location associated to said set of electrograms signals with the mapping of points; step of computing a risk value indicating whether said patient's heart presents a heart rhythm disorder at said spatial location, wherein said risk value is computed from one or more of said electrograms signals and from the value of the parameter and/or the classification associated to at least one point of the mapping of points located at or around said registered spatial location.

## Description

The present invention relates to the field of computer assisted medical diagnosis. More particularly, the invention relates to a computer-implemented method for the quantification of a risk of heart rhythm disorder of a patient, possibly leading to a sudden cardiac death.

Cardiomyopathies might cause a malfunction of the cardiac conduction system, leading to abnormal heart rhythms or cardiac arrhythmias, as ventricular tachycardia (VT), or atrial fibrillation (AF). The scar from a cardiomyopathy, for instance from a previous heart attack, might form abnormal electrical circuits within the heart, which causes VT. Such arrythmias might cause sudden cardiac death, also named SCD. Several treatments, drug-based or involving surgery, might be considered, depending on the seriousness of the arrythmia.

For instance, it is known to treat arrhythmias with an invasive catheter-based ablation. This ablation consists of inserting a catheter into the heart through veins or arteries to burn an area of the heart tissue that is causing arrhythmias. This procedure requires an assessment of the cardiac region contributing to the arrhythmia and subsequently cauterizing a region of cardiac muscle to disrupt the arrhythmia. Even if this procedure is commonly used, it carries risks due to its invasive nature, its complexity, and its length.

For patients presenting a high risk for SCD, an implantable cardioverter-defibrillator, also named ICD, can be used to prevent arrythmias. However, identification of patients admissible to ICD is crucial, since the placement of an ICD involves many risks, as surgical complications, and false shocks.

Heart rhythm disorders are usually diagnosed by a cardiologist from an analysis of electrocardiogram signals (ECGs) acquired from electrodes positioned on the patient's chest or arranged on a catheter inserted into the patient's heart during an electrophysiological mapping. Since ECGs are provided from local electrodes, it might not provide a complete picture of the patient's heart health, even when they are provided from an electrophysiological mapping. Moreover, their interpretation depends on the expertise of the cardiologist and might lead to incorrect readings and false positives or negatives.

Considering the above, there is therefore a need for a method for the estimation of a risk of heart rhythm disorder in a patient's heart, helping a surgeon to accurately determine the most recommended treatment with circumventing the disadvantages of the known solutions.

The object of the present invention is to answer to this need.

For this purpose, the subject of the invention is a computer-implemented method for the estimation of a risk of heart rhythm disorder in a patient's heart, the method comprising:
a. step of receiving at least one mapping of points representing said tissue of said patient's heart, each point being labelled with at least a value of at least one parameter and/or a classification indicating a local characteristic of said tissue at or around said point position;
b. step of receiving a set of electrograms signals, said set of electrograms signals being associated to a spatial location of said patient's heart;
c. step of registering said spatial location associated to said set of electrograms signals with the mapping of points;
d. step of computing a risk value indicating whether said patient's heart presents a heart rhythm disorder at said spatial location, wherein said risk value is computed from one or more of said electrograms signals and from the value of the parameter and/or the classification associated to at least one point of the mapping of points located at or around said registered spatial location.

According to the invention, a mapping of points of a tissue of a patient's heart is supplied. This mapping of points might result from one or more imaging and/or mapping techniques of the tissue, from which a 3D representation of all or part of the tissue is obtained. The 3D representation is associated to local features which are representative, directly, or indirectly and partly, or fully, of the presence or the absence of arrythmias. These local features might be physiological, geometrical, electrical and/or structural characteristic, resulting directly from computing on the mapping of points and/or from another data acquisition technique.

An example of local characteristic might be the thickness of the tissue, for instance determined with measuring the distance separating closest points of inner and outer surface of the tissue. In the case of a wall of the heart, as the myocardium, the thickness of the wall may help to identify areas responsible of arrythmias, such as chamber's wall segment particularly thin or morphological isthmuses, i.e., areas having a thickness lowerthan the thickness of adjacent zones. In particular, such isthmuses might create electrical pathway problems on the heart conduction system, such as re-entry circuits.

An example of classification might be the cellular composition of the tissue. For example, in the case of some cardiac diseases, part of the muscular tissue is replaced by adipocytes or by calcified structures; such structural changes tend to weaken the thickness of the heart as well and might be responsible of arrhythmias.

Simultaneously or sequentially, a set of electrograms signals is supplied. Said electrograms signals might be real-time electrocardiogram signals, each originating from at least one of a plurality of electrodes of a mapping catheter being guided into the patient's heart during an electrophysiological mapping. The set of electrograms signals will then be associated to the spatial location of the mapping catheter into the heart, at the time when said real-time electrograms signals have been captured by the electrodes of the catheter.

As a variant, said electrograms signals might be acquired by a device worn by the patient, as a 12 lead-ECG device placed on the chest, an electrode vest, a smart watch or any suitable wearable device equipped with sensors able to capture and record an electrical activity of the heart. Said spatial location of the electrogram signals might be assumed, if the device is placed at a predetermined location, or acquired by the wearable device or by another device, as a scanner camera or a sensing device associated to the wearable device.

Following the reception of both mapping of points and set of electrograms signals, said set of electrograms signals are registered with the mapping of points. Said registration step might be a registration of the spatial location within the reference frame with which said mapping of points has been captured, a registration of the mapping of points in a reference frame centered on said spatial location, or a registration of both the mapping of points and the spatial location on a common reference frame, as a reference frame of a navigation system.

As a consequence of the registration step, local information on a given part of the patient's heart, obtained from a medical imaging modality and from an electrocardiographic modality and each representative of the patient's heart health, can be combined to determine a risk value, as a probability, indicating whether said patient's heart presents a heart rhythm disorder at said spatial location. Said risk value might be computed with data processing algorithm and/or with machine learning algorithm, implemented on imaging data only, on electrogram data only or a combination of both imaging and electrogram data. Said data might be enhanced with further patient's data, as his age, his gender, his height, his weight, and its medical history.

In the context of the present specification, unless expressly provided otherwise, a "computer" may refer, but is not limited to, a desktop computer, a laptop computer, a tablet computer, a piece of testing equipment, a network appliance, a controller, a digital signal processor, a computational engine within an appliance, a consumer-electronic device, a portable computing device, and/or another electronic device, and/or any combination thereof appropriate to the relevant task at hand. Moreover, the method steps might be all executed on a same device. As a variant, the method steps might be executed on several devices, connected by wires or by a wireless connection.

In the context of the present specification, unless expressly provided otherwise, a "mapping of points of a tissue" is a set of points digitally representing this tissue, said points can be encoded computationally by sequences or lists of numerical values, in particular sequences of at least one numerical value representing the coordinates of said points in a particular reference. The coordinates can be specified in any type of chart, for instance in cartesian, spherical cylindrical coordinates or any other type of geometrical chart in one, two or three spatial dimensions. A mapping of points might be 2D mapping of points or a 3D mapping of points.

A 3D mapping of points might be a 3D model of the heart, namely one or more structured mesh, each comprising a plurality of vertices connected with each otherto define a plurality of faces and/or volumes which approximate internal and/or external surfaces and/or volumes of all or part of the heart. A 3D model might be a triangle mesh or a quadrilateral mesh, or more generally a polygonal mesh, which comprises a plurality of vertices, with edges connecting pairs of vertices and/or polygonal faces connecting a closed set of vertices. As a variant, a 3D model might be a tetrahedral mesh or a hexahedral mesh or a prism mesh, or more generally a volume mesh, which comprises a plurality of vertices, with polyhedrons connecting a closed set of polygons defined by closed set of vertices. Preferably, each vertex and/or edge and/or polygon and/or polyhedron might be labelled as being a part of a specific sub-part of the heart. A 3D model might be stored as a list of vertices each associated with spatial coordinates and with a set of connections to other vertices, or as a list of vertices associated with spatial coordinates and a list of polygons or faces each defined by a subset of vertices contained in said list of vertices, being understood that any other suitable method of storage might be used in the context of the present specification.

A 3D mapping of points might be an unstructured set of points, namely a point cloud, where each point might be or not connected to another point of the point cloud.

In the context of the present specification, said "3D mapping of points" might be (or might be computed from) one of, or a combination of two or more of : an electrophysiological map, resulting from an electrocardiogram (ECG) and/or from an invasive mapping, an anatomical and/or a functional map, resulting from a computed tomography(CT)-scan, a spectral computed tomography(SCT)-scan, a positron emission tomography(PET)-scan, a single photon emission computed tomography(SPECT)-scan, a photon-counting computed tomography(PCCT)-scan or a magnetic resonance imaging(MRI)-scan, and an electro-anatomical map, resulting from an invasive mapping.

In the context of the present specification, unless expressly provided otherwise, "a parameter indicating a local characteristic of said tissue at a point position or around a point position" might be an electrical, a physiological, a physical or a geometrical feature of an area of the tissue whose location corresponds to said position. In the context of a heart tissue as the myocardium, an electrical feature might be an activation time, an electric potential, or a conduction velocity. A physiological feature might be a tissue density. A physical or a geometrical feature might be a tissue thickness, such as a myocardial thickness. It is understood that any other suitable local feature of the heart, which is representative, directly, or indirectly and partly, or fully, of the presence or the absence of arrythmias, might be used in the context of the present specification.

According to one embodiment, said parameter value associated to a point of said mapping of points indicates a local thickness of said tissue at or around said point position.

In the context of the present specification, unless expressly provided otherwise, a "classification indicating a local characteristic of a tissue at a point position or around a point position" might be a class indicating the type of cells composing the tissue at or around a location corresponding said position and/or a class indicating the major type of cells composing the tissue at or around a location corresponding said position and/or a class indicating the proportion of the types of cells composing the tissue at or around a location corresponding said position. In the context of a heart tissue as the myocardium, said type of tissue might be a muscle tissue, a fat tissue, or a calcified tissue. As a variant, a "classification indicating a local characteristic of a tissue at a point position or around a point position" might be a label previously attributed to a point and indicated whether a part of the tissue at or around said point position belongs to a cardiomyopathy scar or a myocardial fibrosis.

According to one embodiment, said classification associated to a point of said mapping of points indicates the presence of a predetermined type of tissue in said tissue at or around said point position. For instance, said classification associated to a point of said mapping of points indicates the presence of fat, calcification and/or muscle in said tissue at or around said point position.

According to a first embodiment, the mapping of points comprises at least a mesh of a wall of the heart, said mesh being generated from one or more images of the heart, each vertex of said mesh is associated with at least one value of a local characteristic of the wall at the heart's point corresponding to this vertex and the segmentation step comprises the selection of a plurality of vertices of said mesh from their associated values, the segmented region being determined from said selected plurality of vertices.

According to one embodiment, the method comprises a preliminary step of acquiring a 3D image and/or recording a 3D image of a patient's heart or a region of his heart, the mesh being computed from said 3D image. The 3D image may be acquired directly from an image acquisition device such as a CT-scan or MRI. Alternatively, the 3D image may be obtained from a recording medium on which it is stored, such as a local memory or a distant database, the 3D image having been acquired beforehand the method.

For example, the step of acquiring the 3D image can be performed by tomography. These techniques are also identified as CT-scan, SCT-scan, PCCT-scan, PET-scan, SPECT-scan, or CAT-scan and are based on the measurement of X-ray absorption by the tissues of an organ. Tomography provides a plurality of 2D images each representing a slice of the organ, which are then combined to reconstruct the 3D image of the anatomical structure of the observed organ. The 3D image comprises a volumetric distribution of pixels, or voxels. Two or more 3D images might be acquired from distinct tomography techniques, or modalities, to build then a multimodal and/or a multidimensional 3D image.

The method according to the invention can thus comprise a 3D modeling step of the 3D image of the heart to generate a 3D model forming then the mapping of points. The 3D modelling step comprises a modeling step of at least one layer, or one wall of the heart shown in the 3D image, such as an inner face and an outer face of the layer of the heart. Said 3D model might be a mesh, especially a polygonal or a polyhedron mesh, of said layer, especially of said inner and outer faces.

According to the invention, a thickness value at a point position or around a point position might be determined with computing the distance between the inner face and the outer face at said position. A classification of the type of tissue at a point position or around a point position might be determined with considering the Hounsfield units encoding the radiodensity at said position.

According to an embodiment, said step of risk value computing comprises a step of computing, from said set of electrograms signals, a probability indicating whether said patient's heart presents a heart rhythm disorder; said risk value being estimated from said probability.

According to an embodiment, said step of risk value computing comprises:
a. a sub-step of extracting at least one timewise feature and/or one morphological feature from said one or more of said electrograms signals; and
b. a sub-step of implementing at least one trained machine learning algorithm on said at least one extracted feature to estimate a first probability indicating whether said patient's heart presents a heart rhythm disorder at said registered spatial location, wherein said trained machine learning algorithm have been trained to estimate, from a value of said at least one predetermined timewise and/or morphological feature of one or more electrograms signals, a probability indicating whether said patient's heart presents a heart rhythm disorder, and wherein said risk value is computed from said first probability.

Priorly to said timewise and/or morphological feature extraction sub-step, said set of electrograms signals might be denoised, for instance with implementing one or more signal processing algorithms based on a low-pass filter and/or a wavelet transform, and/or might be split into elementary electrograms signals with a same duration, said timewise and/or morphological feature extraction sub-step being then implemented on one or more denoised and/or split electrograms signals.

In the context of the present specification, unless expressly provided otherwise, "a timewise feature of an electrogram signal" is a feature which is intrinsically related to the time domain or frequency domain analysis of the electrogram signals.

According to an embodiment, said timewise feature extracting sub-step comprises the estimation of a value of a length cycle from said one or more of said electrograms signals. Said value of a length cycle might be a time interval between two successive R Peaks in the electrogram signal which has the highest amplitude among all the electrograms signals.

For instance, said value of a length cycle might be estimated with firstly measuring a normalized autocorrelation function of said electrogram signal and with estimating a local extremum of said normalized autocorrelation. For example, said normalized autocorrelation function can be estimated by computing the scalar product of a first vector comprising the T first data of the electrogram signal and a second vector comprising the T last data of the electrogram signal, divided by the product of the Euclidean norms of the first vector and the second vector. By varying the number of data T, a maximum and a minimum value of the normalized autocorrelation function can be determined, and then the value selected for the length cycle is chosen as that for which the value of T provides the first local extremum for which the normalized autocorrelation function calculated with this value of T is greater than a set threshold, as for example the difference between the maximum value subtracted by 0.3 times the difference between the maximum value and the minimum value.

As a second example, said value of a length cycle might be estimated with firstly measuring a normalized mean square error function of said electrogram signal and with estimating a local extremum of said normalized mean square error function. For instance, said normalized mean square error function might be estimated by computing for each value of T the Euclidean norm squared by the difference between the first vector and the second vector, divided by the product of the largest absolute value in the first vector by the largest absolute value in the second vector. A maximum and a minimum value of the normalized mean square error function are determined, and then the value selected for the length cycle is chosen as the one for which the value of T provides the first local extremum for which the normalized mean square error function calculated with this value of T is smaller than a set threshold, as for example the difference between the minimum value added by 0.2 times the difference between the maximum value and the minimum value.

Alternatively, or cumulatively, said timewise feature extracting sub-step comprises the estimation of a value of the frequency of said one or more of said electrograms signals, for instance within the FFT of said one or more of said electrograms signals, and/or the amplitude of said one or more of said electrograms signals.

In the context of the present specification, unless expressly provided otherwise, "a morphological feature of an electrogram signal" is a feature which is intrinsically related to morphological analysis of the signal, i.e., analysis based on or related to mathematical morphology.

According to an embodiment, said morphological feature extracting sub-step comprises the estimation of a norm of said one or more of said electrograms signals. For instance, said norm might be the Euclidean norm of the electrogram signal and/or the integrated absolute derivative of the electrogram signal, namely the H10 norm.

In an embodiment, said trained machine learning algorithm might be a boosting-based machine learning algorithm comprising as many input nodes, as timewise and/or morphological features whose values are extracted in the value extraction sub-step.

As a non-limitative example, said boosting-based machine learning algorithm might be a gradient boosting-based machine learning algorithm comprising six input nodes, to which is supplied an input vector comprising the values of the following timewise and/or morphological features, said values being extracted from the electrogram signal which has the highest amplitude among all the electrograms signals : a first cycle length estimation, a second cycle length estimation, the frequency, the amplitude, the Euclidean norm and the integrated absolute derivative of the electrogram signal. The number of considered timewise and/or morphological features, the number of input nodes, the number of terminal nodes and the tree size might be adapted in any appropriate manner without departing from the scope of the disclosure.

In an embodiment, the method comprises a preliminary training step of the boosting-based machine learning algorithm, using a training dataset consisting of a plurality of training vectors, each training vector being associated to a patient and comprising patient's electrograms signals which has been labelled with an indication whether said patient suffers from heart rhythm disorder. Accordingly, in said training step, values of timewise and/or morphological features are extracted from electrograms signals of each training vector and supplied to the boosting-based machine learning algorithm, which predicts a risk of heart rhythm disorder for the associated patient. The error between the prediction and the indication labelling the training vector is computed, and the nodes weights are adjusted using gradient boosting backpropagation of the error to minimize a cost function.

In the above example, the training dataset might comprise 275000 electrograms signals labelled with a value indicating whether the corresponding patient's heart has suffered from a heart rhythm disorder. Values of the six timewise and/or morphological features have been extracted from each of these electrograms signals and supplied to the gradient boosting-based machine learning algorithm for its training, using a logistic loss as cost function.

According to another embodiment, said step of risk value computing comprises a sub-step of implementing said trained machine learning algorithm on said set of electrograms signals to estimate a first probability indicating whether said patient's heart presents a heart rhythm disorder at said registered spatial location, wherein said trained machine learning algorithm have been trained to estimate, from a set of electrograms signals, a probability indicating whether said patient's heart presents a heart rhythm disorder, and wherein said risk value is computed from said first probability.

In an embodiment, said trained machine learning algorithm might comprise at least a first stage configured to provide, from said set of electrograms signals, a plurality of feature maps and a second stage configured to estimate, from said features maps, said first probability. Said first stage might be a convolutional neural network and said second stage might be a fully connected neural network.

As a non-limitative example, said convolution neural network might comprise a first convolution layer configured to extract, from a set of 16 electrograms signals provided as input to the convolution neural network, 200 features, followed by a second convolution layer which extracts 3000 features from the first layer, a third convolution layer which extracts 20000 features from the second layer, a fourth convolution layer which extracts 8000 features from a max pooling layer following the third layer, and a fifth convolution layer which extracts 1000 features from the second layer.

According to this example, the fifth convolution layer is linked to a fully connected neural network, forming said second stage and which comprises two fully connected layers comprising respectively 50 and 10 nodes, an array of probabilities being provided from the last fully connected layer. The number of convolution layers, the number of nodes of each convolution layer, the size of each filter, the type of pooling function and the type of activation function of the first stage, and the number of fully connected layers, the number of nodes of each fully connected layer and the type of activation function of the second stage might be adapted in any appropriate manner without departing from the scope of the disclosure.

In this example, the training of this convolutional neural network might made using by using 275000 electrogram signals labelled with a value indicating whether the corresponding patient's heart has suffered from a heart rhythm disorder. The training is using a binary cross entropy loss.

According to an embodiment, the method comprises:
a. a step of receiving a plurality of sets of electrograms signals, each set of electrograms signals being associated to a spatial location of said patient's heart;
b. a step of modelling an electrocardiographic mapping of the patient's heart from the plurality of sets of electrograms signals; each point of said electrocardiographic mapping being associated with at least one value determined from the set of electrograms signals associated to the spatial location corresponding to said point;
c. a step of registering said electrocardiographic mapping with the mapping of points.

Said plurality of sets of electrograms signals might have been collected simultaneously, each set being issued from an electrocardiographic signal acquisition device positioned on a specific location of the patient's chest. Said electrocardiographic signal acquisition devices might form together an electrocardiographic vest. Said specific locations might be predetermined locations, corresponding to predefined anatomical landmarks. As a variant, said specific locations might be captured using a specific device, as a camera.

As a variant, said plurality of sets of electrograms signals might have been collected sequentially, each set being issued from a plurality of electrodes of a mapping catheter which has being guided into the patient's heart during an electrophysiological mapping.

Considering said embodiment, said step of risk value computing might be implemented for each point of said mapping of points, for each point of said electrocardiographic mapping, or for one point or for a group of points only of said mapping of points and/or said electrocardiographic mapping.

Said value determined from the set of electrograms signals associated to a spatial location corresponding to a point of the electrocardiographic mapping might a timewise feature and/or a morphological features extracted from said set of electrograms signals, and/or a first probability, determined from said set of electrograms signals, indicating whether said patient's heart presents a heart rhythm disorder at said spatial location.

As a non-limitative example, said registration step might comprises the computing of a spatial transformation which, when applied to one model among the electrocardiographic mapping and the mapping of points, minimizes a predefined error metric between the result of this spatial transformation on this model and the other model.

For instance, said registration step might comprises the segmentation, on each of the electrocardiographic mapping and the mapping of points, of a same predetermined known area of the heart, and the computing of a spatial transformation which, when applied to one model among the electrocardiographic mapping and the mapping of points, minimizes a predefined error metric between the result of this spatial transformation on a group of points of said model which corresponds to said segmented predetermined known area and a group of points of the other model which corresponds to said segmented predetermined known area.

According to an embodiment, said step of risk value computing comprises a sub-step of adjusting the first probability following the value of the parameter and/or the classification associated to at least one point of the mapping of points located at or around said registered spatial location.

As an example, wherein said parameter indicating a local characteristic of said tissue is a local thickness of said tissue, said risk value is determined with computing a linear /or logarithmic combination of the first probability and of a value computed from the thicknesses of a plurality of points of the mapping of points located at or around said registered spatial location. For instance, said risk value might correspond to a norm of a gradient of the thicknesses of said plurality of points, weighted with the first probability. Said plurality of points might be a plurality of points forming a region with predetermined dimensions and centered on said registered spatial location.

As another example, said first probability might be retained as the risk value upon a comparison of the thicknesses of a plurality of points of the mapping of points located at or around said registered spatial location to a predetermined threshold. For instance, said first probability is retained as the risk value whether a norm of a gradient of the thicknesses of a plurality of points of the mapping of points located at or around said registered spatial location is greater than a predetermined threshold, and is null otherwise.

According to another embodiment, said step of risk value computing comprises:
a. a sub-step of estimating, from the value of the parameter and/or the classification associated to at least one point of the mapping of points located at or around said registered spatial location, a second probability indicating whether said patient's heart presents a heart rhythm disorder at said spatial location,
b. a sub-step of computing a third probability indicating whether said patient's heart presents a heart rhythm disorder with combining said first and second probabilities.

In this embodiment, the second probability might be computed priorly to the first probability computing, following a medical imaging procedure at the end of which second probabilities are computed for a plurality of points of one or more regions of interest of said mapping of points to identify potential ablation targets. Said first probability might be then computed for each potential ablation targets, following an electrophysiological mapping in which a mapping catheter is moved to or close to said potential ablation target, said first probability being then computed and said first and second probabilities being then combined to confirm whether said potential ablation target can be considered as relevant.

As another example, the second probability might be computed concomitantly or successively to the first probability computing. For instance, an invasive or non-invasive electrocardiographic mapping, comprising the collection of electrograms signals for a plurality of areas of the patient's heart, might be built for the computing of a set of first probabilities each associated to one of these areas. The electrocardiographic mapping can be mapped on the mapping of points resulting from a medical imaging procedure. A set of second probabilities can be computed for all the points of the mapping of points, the set of first and second probabilities being then combined in a set of third probabilities, considering the registration of the electrocardiographic mapping on the mapping of points. As a variant, a set of second probabilities can be computed only for points of the mapping of points belonging to one ore more regions of interest of the patient's heart, segmented according to the parameter values and/or the classifications. A set of third probabilities might be then computed with combining said set of second probabilities and the first probabilities associated to the heart's areas corresponding to the segmented regions of interest. As a further variant, some only of the heart's areas might be selected, considering their associated first probabilities and a set of second probabilities can be computed only for points of the mapping of points corresponding to these selected areas. A set of third probabilities might be then computed with combining said set of first and second probabilities.

Said third probability might be computed with multiplying the first probability with the second probability, with computing any linear /or logarithmic combinations of the first and second probabilities, or with retaining the higher or the lower value among the first and second probabilities as the third probability.

According to an embodiment, the second probability estimation sub-step comprises:
a. a sub-step of extracting, from a plurality of points of the mapping of points defining a region of interest located at or around said registered spatial location, a value of at least one predetermined morphological feature of said region of interest;
b. a sub-step of implementing a second trained machine learning algorithm on said extracted value to estimate said second probability, wherein said second trained machine learning algorithm have been trained to estimate, from a value of said at least one morphological feature of a region of interest of a patient's heart, a probability indicating whether said patient's heart presents a heart rhythm disorder.

Preferably, the second probability estimation sub-step might comprise a sub-step of segmentation of the mapping of points from said parameter values and/or from said classifications to identify a region of interest of a predetermined kind, which matches a predefined electrical conductivity criterion, and which is located at or around said registered spatial location.

In the context of the present specification, unless expressly provided otherwise, "an area of a tissue matching a predefined electrical conductivity criterion" might be any area of the tissue having an electrical conductivity lower than a given threshold and/or being defined by a border corresponding to a local gradient of electrical conductivity greater than a given threshold.

For instance, said kind of region of interest might be:
a. a zone in which the electrical conduction is slowed down, as a myocardial isthmus having an electrical gradient which has the consequence of inducing a slow down of the electrical conduction;
b. an arrhythmogenic zone, as myocardium scar in which the electrical circulation is sustained according to a rhythm different from the cardiac sinus rhythm;
c. a hypertrophic segment of a wall of heart's chamber, as a segment of the left ventricle myocardium;
d. an apical aneurysm or a thin-walled segment of the most distal portion of the left ventricle myocardium.

Said kind of region of interest might be manually defined by the cardiologist and/or automatically determined, prior to the implementation of the method and according to the patient's medical history.

According to one embodiment, said segmentation step comprises a direct thresholding of the parameter values of a plurality of points of said mapping of points located at or around said registered spatial location, for instance following a comparison of their thickness to a thickness level, for example of 1mm, 2mm, 3mm, 4mm or 5mm.

According to a second embodiment, said segmentation step comprises the segmentation of the mapping of points from said parameter values and/or from said classifications to identify at least a couple of areas of interest in said mapping of points which are located at or around said registered spatial location, each area of interest corresponding to an area of said tissue matching a predefined electrical conductivity criterion, wherein the points of the mapping of points extending between said couple of areas of interest define together said region of interest whether their parameter values is lower than the parameter values of said couple of areas of interest.

For instance, said segmentation step might comprise an iterative decrease of an electrical conductivity threshold; and, for each iterative value of said electrical conductivity threshold, an identification step of two or more groups of points of said mapping of points located at or around said registered spatial location, wherein each group of points is selected from their parameter values and/or from their classifications so as to define an area of said tissue having an electrical conductivity lower than said iterative value of said electrical conductivity threshold, said iterative decrease of the electrical conductivity threshold being stopped when the border of each corresponding groups of points remains globally identical from the previous iteration to the current iteration, said corresponding groups of points thus defining said areas of interest. For instance, each area of interest is progressively identified with decreasing a thickness threshold until the area remains the same, meaning that this area has globally a same thickness, that the decreasing step required to further change the shape of this area is high and that the border of this area corresponds to a local gradient of electrical conductivity greater than a given threshold.

According to a third embodiment, said segmentation step might be implemented from any suitable region growing method, with considering points located at or around said registered spatial location as seed points and recursively considering neighbors of these points with estimating whether they should be considered as part of a region of interest upon their parameter values.

Whatever the embodiment considered, a region of interest identified within the mapping of points might be modified, for instance with smoothing or eroding its border.

According to an embodiment in which said kind of region of interest is an isthmus, said value extraction sub-step might comprise the computing of a value of one or more, or a combination, of the following features:
a. a thickness of the isthmus, said thickness being an absolute thickness and/or a relative thickness compared to the thicknesses of the two adjacent areas to the isthmus;
b. a thickness gradient at the boundary of the isthmus and one adjacent area to the isthmus;
c. a width of the isthmus, considering the spacing of said adjacent areas;
d. a length of the isthmus, considering the arrangement of adjacent areas;
e. a ratio between the width of the isthmus and the length of the isthmus;
f. a norm or a metric indicating the evolution of the width and/or of the thickness of the isthmus along its length;
g. the topology of the entrance and/or the exit of the isthmus, such as the curvature of the funnel formed by the two adjacent areas;
h. a thickness gradient at the entrance and/or the exit of the isthmus;
i. a metric indicating the quantity of fat tissue and/or calcification tissue in the isthmus, such as a volume density of fat tissue and/or calcification tissue in the isthmus.

According to another embodiment in which said kind of region of interest is a scar, said value extraction sub-step might comprise the computing of a value of one or more, or a combination, of the following features:
a. a compactness of the scar, such as the compactness of the scar evaluated for several threshold thickness values;
b. a number of connected components of the scar, such as the number of connected components of the scar evaluated for several threshold thickness values;
c. a metric related to the thickness spatial distribution of the scar, such as a spatial thickness gradient of the whole scar or a thickness gradient at the scar's border;
d. a metric indicating the quantity of fat tissue and/or calcification tissue in the scar, such as a volume density of fat tissue and/or calcification tissue in the scar, a metric indicating a spatial distribution of fat tissue and/or calcification tissue in the scar, a compactness of fat tissue and/or calcification tissue in the scar;
e. a metric related to the extent of myocardial fibrosis, such as a ratio between the volume of the scar and the volume of the chamber containing the scar, namely the left ventricle.

In an embodiment, said trained machine learning algorithm might be a densely connected neural network comprising at least one input layer comprising as many input nodes, or artificial neurons, as predetermined morphological features whose values are extracted in the value extraction sub-step, one or more dense hidden layers each comprising a plurality of nodes, and an output layer comprising a single output node from which said risk value is provided.

As a non-limitative example, the input layer might comprise 31 input nodes, to which is supplied an input vector comprising the extracted values, namely the mean, min and max values of the isthmus's absolute thickness; the isthmus's relative thickness; the thickness gradient at the boundary of the isthmus; the isthmus's width, the isthmus's length, the isthmus's width over length ratio; the curvature of the entrance and the exit of the isthmus; and the thickness gradient at the entrance and the exit of the isthmus, and the volume density of fat tissue and of calcification tissue in the isthmus.

In this example, the densely connected neural network might comprise a first hidden layer comprising 25 nodes and a second hidden layer comprising 25 nodes. Each node of the first hidden layer is fully connected to each input node of the input layer, each node of the second hidden layer is fully connected to each node of the first hidden layer and the single output node of the output layer is fully connected to each node of the second hidden layer. Each node of the hidden layers might be configured with a rectified linear activation function ReLU, and the single node of the output layer might be configured with a logistic function. The number of considered morphological features, the number of input nodes of the input layer, the number of hidden layers, the number of nodes of each hidden layer and the activation function associated to each node might be adapted in any appropriate manner without departing from the scope of the disclosure.

In an embodiment, the method comprises a preliminary training step of the densely connected neural network, using a training dataset consisting of a plurality of training vectors, each training vector being associated to a patient and comprising a mapping of points of the patient's heart labelled with an indication whether said patient suffers from heart rhythm disorder. Accordingly, in said training step, at least one region of interest is segmented from the mapping of points of each training vector, and values of predetermined morphological feature are extracted from said region of interest and supplied to the densely connected neural network, which predicts a risk of heart rhythm disorder for the associated patient. The error between the prediction and the indication labelling the training vector is computed, and the synaptic weights and the biases of the nodes of the layers are adjusted using gradient backpropagation of the error to minimize a cost function.

In the above example, the training dataset might comprise cardiac data aggregated for 500 patients, from which a plurality has a heart which does not suffer from arrythmia, another plurality has a heart which suffers from arrythmia although the arrythmia has not causes a SCD and another plurality has a heart suffering from arrythmia which has caused a SCD. For each patient, values of the 31 above-mentioned morphological features of at least an isthmus have been extracted, while the corresponding mapping of points labelled with an indication whether said patient's heart has suffered from SCD. A batch gradient descent is used for the training step and the weights of the layers are adjusted until a mean square error is less than 10-5. The amount of data of the training dataset, the type of data used in the training dataset, the method of gradient backpropagation and the function cost might be adapted in any appropriate manner without departing from the scope of the disclosure.

According to another embodiment, said parameter values and/or said classifications associated to the points forming said segmented region of interest form together a parameter and/or classification map of said region of interest; and wherein the risk value computing step comprises a sub-step of implementing said trained machine learning algorithm on said parameter and/or classification map to estimate said risk value, wherein said trained machine learning algorithm have been trained to estimate, from a parameter and/or classification map of a region of interest of a patient's heart, a probability indicating whether said patient's heart presents a heart rhythm disorder.

In an embodiment, said trained machine learning algorithm might comprise at least a first stage configured to provide, from said parameter and/or classification map, a plurality of feature maps and a second stage configured to estimate, from said features maps, said risk value.

Said first stage might be a graph convolution neural network (GCNN) or a convolution neural network (CNN) comprising a contracting pathway receiving said parameter and/or classification map as an input and being configured to extract features from said parameter and/or classification map. For instance, said contracting pathway might comprise one or more image or graph convolution layers, wherein each convolution layer processes its input one or more convolution filters or kernels, each convolution layer being associated to an activation layer. If necessary, said contracting pathway might also comprise one or more pooling layers, and a final flattening layer from which is provided a one-dimensional vector containing said extracted features.

As a non-limitative example, said graph convolution neural network might comprise two consecutive graph convolution layers, followed by a pooling layer, followed again by three further graph convolution layers and a final flattening layer. The two first graph convolution layers might each comprise 5000 nodes, a 64x64 filter and a rectified linear activation function ReLU, the two second graph convolution layers might each comprise 500 nodes, a 64x64 filter and a rectified linear activation function ReLU, while the last comprises 500 nodes and a 1x1 filter. The pooling layer might be a max pooling layer. The number of convolution layers and pooling layer, the number of nodes of each convolution layer, the size of each filter, the type of pooling function and the type of activation function might be adapted in any appropriate manner without departing from the scope of the disclosure.

The second stage might be a fully connected neural network comprising a succession of fully connected layers, the first of which being an input layer receiving said one-dimensional vector provided from the graph convolution neural network, and the last of which being an output layer comprising a single output node from which said risk value is provided.

In the above-mentioned example, said fully connected neural network might comprise a first layer of 128 nodes, a second layer of 64 nodes, a third layer of 32 nodes and a last layer of a single node. Each node of the three first layers might be configured with a rectified linear activation function ReLU, and the single node of the last layer might be configured with a logistic function. The number of nodes of each layer and the activation function associated to each node might be adapted in any appropriate manner without departing from the scope of the disclosure.

In an embodiment, the method comprises a preliminary training step of both the graph convolution neural network and the fully connected neural network, using a training dataset consisting of a plurality of training vectors, each training vector being associated to a patient and comprising a mapping of points of the patient's heart labelled with an indication whether said patient suffers from heart rhythm disorder. Accordingly, in said training step, at least one region of interest is segmented from the mapping of points of each training vector, and the parameter and/or classification map of said region of interest, either formatted as a picture or as a graph, is supplied to the machine learning algorithm, which predicts a risk of heart rhythm disorder for the associated patient. The error between the prediction and the indication labelling the training vector is computed, and the weights of the filters of the convolution layers and the weights of the fully connected layers are adjusted using gradient backpropagation of the error to minimize a cost function.

In the above example, the training dataset might comprise cardiac data aggregated for 600 patients, from which a plurality has suffers from ventricular tachycardia, ventricular fibrillation, and cardiac arrest. For each patient, a 3D model of his heart has been acquired and segmented to identify a region corresponding to a cardiomyopathy scar, said 3D model being labelled with an indication whether said patient's heart has suffered from SCD. A batch gradient descent is used for the training step and the weights of the layers are adjusted using a binary cross-entropy loss as cost function. The amount of data of the training dataset, the type of data used in the training dataset, the method of gradient backpropagation and the function cost might be adapted in any appropriate manner without departing from the scope of the disclosure.

According to another embodiment, said trained machine learning algorithm might be implemented on a region of interest in said mapping of points corresponding to a hypertrophic segment of a wall of heart's chamber, as the left ventricle myocardium, or to a thin-walled segment of the most distal portion of the left ventricle myocardium, wherein said trained machine learning algorithm have been trained to estimate, from a region of interest of 3D model of patient's heart, a probability indicating whether said region of interest might be responsible of a heart rhythm disorder.

According to an embodiment, the method comprises, further to the step of risk value computing, a step of displaying on a navigation system display device said mapping of points with a landmark at the registered spatial location, wherein a visual feature of said landmark depends on said computed risk value. As a variant, said mapping of points might be replaced, on the navigation system display device, by an electrophysiological mapping or an electrocardiographic imaging. Said visual feature might be a color of the landmark, for instance selected on a graduated color scale, each graduation being associated to a probability range. It is thus possible for the cardiologist to visualize, on a 3D model of a patient's heart, for each point, or for each region of points, the probability of a heart rhythm disorder at a location of the heart corresponding to said point or region of point. It might also be possible, for a surgeon performing an electrophysiological mapping or a non-invasive electrocardiographic imaging, to obtain a visual indication whether a particular region of the heart, which is travelled by its catheter or its imaging ECG device, might cause a heart rhythm disorder.

According to an embodiment, the method comprises, further to the step of risk value computing, a step of determining, from said risk value and from said mapping of points, an ablation target area at or around said registered spatial location. For instance, an ablation target area might be detected whether the risk value associated to a registered spatial location, or a value derived from a group of risk values associated to a plurality of adjacent registered spatial location, is above a predetermined threshold. Said ablation target area might be then visually identified on a navigation system display device on which said mapping of point is displayed.

The subject of the invention is also a computing device for the implementation of the method according to one of the embodiments, said computing device comprising a memory arranged to receive at least one mapping of points and a set of electrograms signals and a computing unit arranged to implement at least the registration step and the risk value computing step from said set of electrograms signals and said mapping of points.

Figure 1 is a logic flow diagram that depicts a computer-implemented method, according to an embodiment of the invention;

Figure 2 shows a schematic representation of a 3D mapping of points representing a tissue of a patient's heart;

Figure 3 shows a schematic representation of a set of electrograms signals and of an electrocardiographic mapping of the patient's heart;

Figure 4 shows a schematic representation of the result of a segmentation step of the method depicted in Figure 1 when implemented on the 3D mapping of points of Figure 2 and morphological features extracted from this result; and

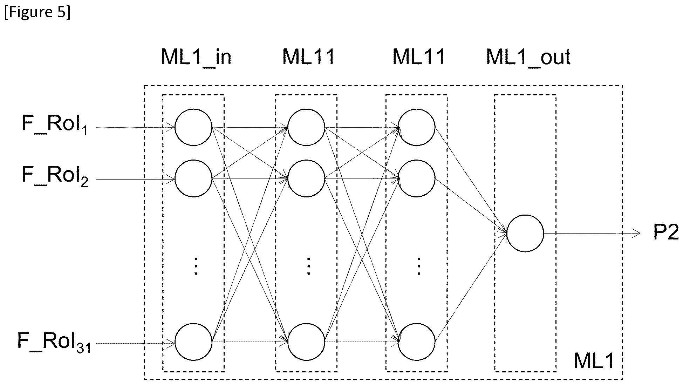

Figure 5 shows a schematic representation of a first machine learning algorithm implemented in the method of Figure 1.

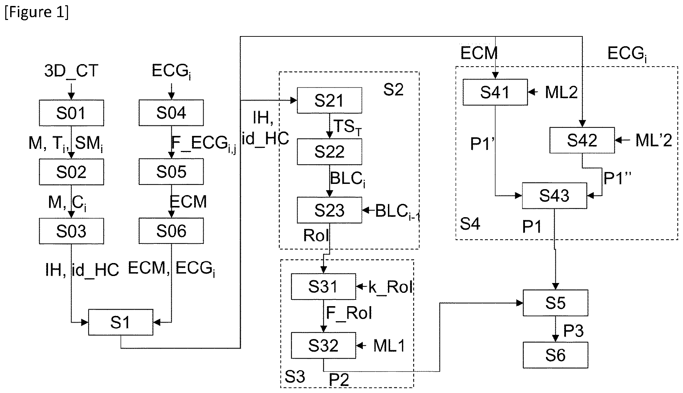

Reference is made to Figure 1 which shows a computer-implemented method for estimation of a risk of heart rhythm disorder in a patient's heart.

In a preliminary step S01, a 3D image 3D_CT of the patient's heart has been acquired from a CT-scan method, and a 3D model IH of the patient's heart has been generated from the 3D image.

More precisely, an inner surface or layer and an outer surface or layer of the patient's heart myocardium have been modelized from the 3D image 3D_CT with defining a plurality of vertices of meshes representing said surfaces. This modeling results in a mesh M of the inner surface or layer or of the outer surface or layer of the myocardium, wherein each vertex Mᵢ of the mesh M is therefore labelled with the value of the thickness Tᵢ of the myocardium, meaning the thickness computed from these inner and outer surfaces or layers, at the location of the heart corresponding to the position of this vertex.

In the depicted example, these vertices have been segmented, according to their thickness. Said mesh M comprises then a plurality of superimposed sub-meshes SMᵢ, with each sub-mesh corresponding to a thickness range of the myocardium.

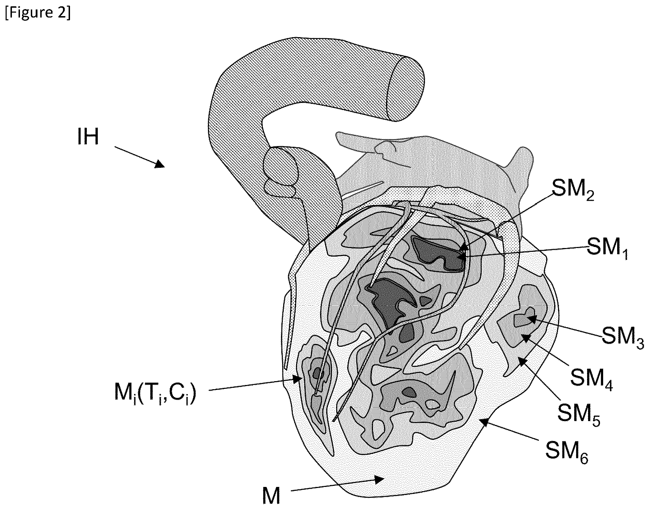

Reference is made to Figure 2 which shows an example of a 3D model IH. The model IH comprises a mesh M comprising six superimposed thickness sub-meshes SM₁ to SM₆ corresponding each to a range of myocardium thickness, namely 5mm to 6mm, 4mm to 5mm, 3mm to 4mm, 2mm to 3mm, 1mm to 2mm, and 0 to 1mm. For description conciseness, regions of greater thickness have been depicted darker than regions of lower thickness.

Moreover, in a further preliminary step S02, each point of the 3D image has been associated to a numerical value in terms of Hounsfield units encoding the radiodensity at said point. Since calcification type cells, fat type cells and muscle type cells do not present a same radiodensity, it is therefore possible to discriminate, for a slice of the myocardium at a defined location of the heart, the proportion of calcification, fat and muscle type cells composing said slice. Each vertex Mᵢ of the mesh M has been therefore labelled with a class Cᵢ indicating the major type of cells composing the myocardium at the location of the heart corresponding to the position of this vertex.

In the depicted example, said class Cᵢ is selected among classes C₁ "calcification tissue", C₂ "fat tissue" and C₃ "muscle tissue".

One can note that the steps S01 to S02 might be executed by another computing unit than the one used for the rest of the method. Moreover, steps S01 to S02 might be executed fully automatically by a computing unit or with a human assistance. The resulting 3D model IH might be stored on a recording medium, such as a memory of the computer or an external memory support or a distant database through which it is accessible to the computer.

In a step S03, the 3D model IH is provided to the computing unit of the computer. A kind of region of interest k_Rol to be segmented is also provided to the computing unit, either manually by a cardiologist or automatically from the patient's medical history. In the depicted example, said kind of region of interest k_Rol is a myocardial isthmus having an electrical gradient which has the consequence of inducing a slow down of the electrical conduction of the heart.

In a preliminary step S04, a plurality of set of electrocardiograms signals ECGᵢ of said patient's heart are received.

In the depicted example, said electrocardiograms signals ECGᵢ are real-time electrocardiogram signals, each originating from a plurality of electrodes of a mapping catheter being guided into said organ during an electrophysiological mapping procedure. Each set of electrocardiograms signals ECGᵢ is therefore associated to a spatial location of the patient's heart at which said electrodes were located when the set was acquired.

As a variant, said plurality of sets of electrocardiograms signals ECGᵢ might have been acquired by an electrocardiographic vest comprising a plurality of electrocardiographic acquisition devices positioned at different locations of the patient's chest.

In the step S04, values of several predetermined timewise and morphological features F_ECG_{i,j} are extracted from each set of electrocardiograms signals ECGᵢ. In the depicted example, for each set ECGᵢ, the following timewise and morphological features F_ECG_{i,1} to F_ECG_{i,6} are extracted from the electrocardiogram signal which has the highest amplitude among all the electrocardiogram signals ECGᵢ : a first cycle length estimation based on the ECG signal autocorrelation, a second cycle length estimation based on the ECG signal mean square error, the frequency, the amplitude, the Euclidean norm and the integrated absolute derivative of the electrocardiogram signal.

In a further preliminary step 505, an electrocardiographic mapping ECM of the patient's heart is modeled from the plurality of sets of electrograms signals ECGᵢ, and their associated spatial locations. Each point ECMᵢ of said electrocardiographic mapping ECM corresponds thus to an area of the patient's heart and is labelled with the values of the timewise and morphological features F_ECG_{i,j} which have been extracted from the set of electrocardiograms signals ECGᵢ associated with the spatial location of this area.

In a step 506, the electrocardiographic mapping ECM and the sets of electrocardiograms signals ECGᵢ are provided to the computing unit of the computer.

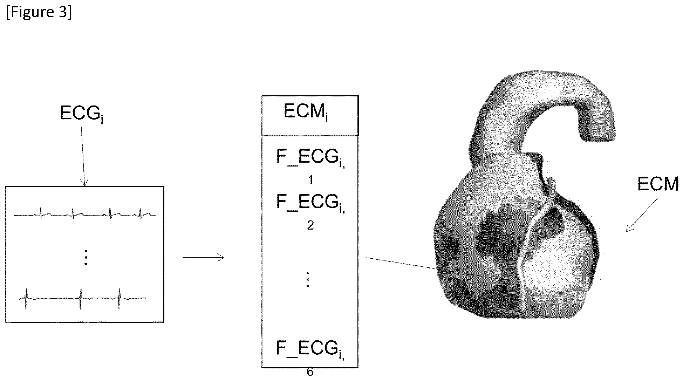

Reference is made to Figure 3 which shows a set of electrocardiograms signals ECGᵢ, extracted timewise and morphological features F_ECG_{i,1} to F_ECG_{i,6}, and the resulting electrocardiographic mapping ECM.

In a step S1, the electrocardiographic mapping ECM is registered with the 3D model IH.

Said registration step S1 comprises the segmentation, on each of the electrocardiographic mapping ECM and the mapping of points IH, of one or more known areas of the patient's heart, as a given heart chamber as the left ventricle, and/or a part of a given blood vessel as the coronary sinus. Said segmentation step is followed with the computing of a spatial transformation which, when applied to the electrocardiographic mapping ECM, minimizes a predefined error metric between the result of this spatial transformation on a group of points of said mapping ECM which corresponds to said segmented predetermined known area and a group of points of the 3D model IH which corresponds to said segmented predetermined known area.

Consequently to said registration step S1, each point of the electrocardiographic mapping ECM can be mapped on a point of the 3D model IH, meaning that, for each area of the patient's heart, an electrocardiographic feature is known from the electrocardiographic mapping ECM and a morphological feature is known from the 3D model IH.

In a step S2, at least one region of interest Rol, which matches a predefined electrical conductivity criterion, is segmented in said 3D model IH.

The segmentation step comprises a segmentation, in the 3D model IH, of a couple of areas of interest BLC, which each matches a predefined electrical conductivity criterion.

In the depicted example, said segmentation step S2 comprises an iterative decrease of an electrical conductivity threshold, being approximated here by a thickness threshold TS_{T}. Said thickness threshold TS_{T} is thus progressively decrease, in a sub-step S21, from a maximum value, namely 6 mm, to a minimum value, namely 1 mm.

For each iterative threshold TS_{T}, in a sub-step S22, connected vertices Mᵢ of the mesh M whose thickness values Tᵢ are above this threshold are selected to define areas BLCᵢ of the myocardium.

At each next iteration on said threshold TS_{T}, in a sub-step S23, the newly defined areas BLCᵢ are compared with the previous defined areas BLCᵢ₋₁. When the border of a newly defined area BLCᵢ remains globally the same than the corresponding previously defined area BLCᵢ₋₁, the iteration is stopped for this area and the set of connected vertices defining said area BLCᵢ is classified as an area of interest BLC.

Each area of interest BLC resulting from the step S23 has then globally a same thickness and its border corresponds to a quick change of thickness toward its adjacent regions, meaning that this border corresponds to a local gradient of electrical conductivity greater than a given threshold. The vertices Mᵢ which extend between this couple of areas of interest BLC form then a region of interest Rol whose kind corresponds to the kind k_Rol, namely a myocardial isthmus.

As an undepicted variant, sub-steps S21 to S23 might be implemented with considering classes Cᵢ rather than thickness values Tᵢ of the vertices, or with considering a combination of these classes and values.

As a further undepicted variant, said sub-steps S21 to S23 might be replaced with a single thresholding of the thickness values Tᵢ of the vertices of the mesh M, for instance following a comparison to a given thickness level, for example of 1mm, 2mm, 3mm, 4mm or 5mm. As another undepicted variant, said segmentation step S2 might be replaced with any suitable region growing method, with considering vertices of the mesh M labelled with thickness values Tᵢ lower than a given threshold as seed points and recursively considering neighbors of these vertices with estimating whether they should be considered as part of a region of interest upon their thickness values Tᵢ.

Reference is made to Figure 3 which shows an example of a couple of areas of interest BLC and the isthmus Rol extending between these areas of interest BLC, resulting from the segmentation of the mesh M according to the step S2.

In a step S3, a probability P2 indicating whether said patient's heart presents a heart rhythm disorder is estimated.

Firstly, in a sub-step S31, values of several morphological features F_Rol are extracted from the region of interest Rol. The types of considered morphological features F_Rol depend on the kind of said region of interest k_Rol.

In the depicted example, since the kind k_Rol of the region of interest Rol is an isthmus, the considered morphological features F_Rol are the isthmus's absolute thickness; the isthmus's relative thickness; the thickness gradient at the boundary of the isthmus; the isthmus's width, the isthmus's length, the isthmus's width over length ratio; the curvature of the entrance and the exit of the isthmus; and the thickness gradient at the entrance and the exit of the isthmus, and the volume density of fat tissue and of calcification tissue in the isthmus.

For each of these features F_Rol, except for the volume density, a mean value, a minimum value, and a maximum value are computed from the vertices Mᵢ of the isthmus Rol and the adjacent areas BLC, from their coordinates and from their thickness values Tᵢ. A value of the volume density is computed from the vertices Mᵢ of the isthmus Rol and from their classes Cᵢ.

In a sub-step S32, the extracted values of the features F_Rol are supplied, as an input vector, to a first trained machine learning algorithm ML1 for the prediction of the probability P2 that the patient's heart presents a heart rhythm disorder.

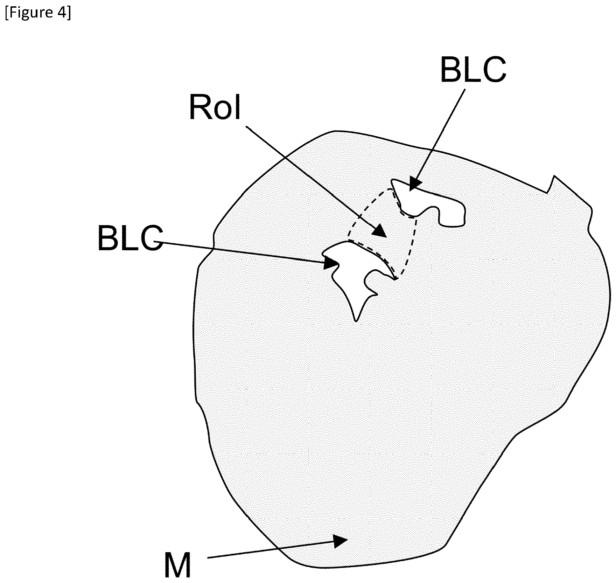

Reference is made to Figure 4 which shows an architecture of an appropriate machine learning algorithm ML1 for the implementation of the sub-step S32.

Said machine learning algorithm ML1 is a densely connected neural network comprising an input layer comprising an input layer ML1_in with 31 input nodes for the reception of the extracted values of the features F_Rol and an output layer ML1_out with a single output node from which the probability P2 is provided.

In this example, the densely connected neural network ML1 comprises a first hidden layer ML11, comprising 25 nodes, and a second hidden layer ML12, comprising 25 nodes. Each node of the first hidden layer ML11 is fully connected to each input node of the input layer ML1_in, each node of the second hidden layer ML12 is fully connected to each node of the first hidden layer ML11 and the single output node of the output layer ML1_out is fully connected to each node of the second hidden layer ML12. Each node of the hidden layers is configured with a rectified linear activation function ReLU, and the single node of the output layer is configured with a logistic function.

The densely connected neural network ML1 has been previously trained with a training dataset to estimate, from a set of values of the morphological features F_Rol, a probability that a patient's heart presents a heart rhythm disorder. In the depicted example, the training dataset comprises heart's 3D models of 500 patients, from which a plurality has a heart which does not suffer from arrythmia, another plurality has a heart which suffers from arrythmia although the arrythmia has not causes a SCD and another plurality has a heart suffering from arrythmia which has caused a SCD. For each patient, values of the 31 above-mentioned morphological features F_Rol have been extracted from its heart's 3D model, while the model has been labelled with an indication whether said patient's heart has suffered from SCD. A batch gradient descent has been used for the training step and the weights of the layers have been adjusted until a mean square error is less than 10-5.

As a variant to sub-steps S31 to S32, a graph formed by the thickness values Tᵢ and the classes Cᵢ of the vertices Mᵢ of the isthmus Rol might be directly supplied to a machine learning algorithm, comprising a graph convolution neural network as a first stage and a fully connected network as a second stage, said machine algorithm being trained to predict said probability P2 from a graph of thickness values and classes.

Only one machine learning algorithm might be used to estimate the first probability, or multiple machine learning algorithms might be used simultaneously to estimate two or more probabilities, which are then combined to estimate the probability P2.

In a step S4, a probability P1, indicating whether said patient's heart presents a heart rhythm disorder, is computed from said set of electrocardiogram signals ECG.

In a sub-step S41, the extracted values of the features F_ECG_{i,j} of the points of the electrocardiographic ECM matching with the points of the isthmus Rol are supplied, as an input vector, to a second trained machine learning algorithm ML2 for the prediction of a probability P1' that the patient's heart presents a heart rhythm disorder.

In the depicted example, the second machine learning algorithm ML2 is a gradient boosting-based machine learning algorithm.

The gradient boosting-based machine learning algorithm ML2 has been previously trained with a training dataset to estimate, from a set of values of timewise and morphological features F_ECG of an electrocardiogram signal, a probability that a patient's heart presents a heart rhythm disorder. In the depicted example, the training dataset comprises 275000 electrocardiogram signals labelled with a value indicating whether the corresponding patient's heart has suffered from a heart rhythm disorder. Values of the six timewise and morphological features have been extracted from each of these electrograms signals and supplied to the gradient boosting-based machine learning algorithm for its training, using a logistic loss as cost function.

The set of electrocardiogram signals ECGᵢ associated to the spatial locations corresponding to the isthmus Rol are directly supplied, in a sub-step S42 to an alternative machine learning algorithm ML'2.

Said trained machine learning algorithm ML'2 comprise a convolutional neural network CNN, as a first stage, trained to provide, from said set of electrocardiogram signals ECG, a plurality of feature maps, and a fully connected neural network, as a second stage, trained to estimate, from said features maps, a probability P1".

In the depicted example, the training of this machine learning algorithm ML'2 is made using by using the 275000 electrogram signals labelled with a value indicating whether the corresponding patient's heart has suffered from a heart rhythm disorder. The training is using a binary cross entropy loss.

As shown in Figure 1, both machine learning algorithms ML2 and ML'2 are used simultaneously to estimate two probabilities P1' and P1", which are then combined in a step S43 to estimate the probability P1. However, only one of these machine learning algorithm ML2 and ML'2 might be used to estimate the probability P1.

In a step S5, the probabilities P1 and P2 are combined to estimate a third probability P3, which might be interpretated by a cardiologist to derive a risk value indicating whether said patient's heart presents a heart rhythm disorder.

Said third probability P3 is computed with multiplying the first probability P1 with the second probability P2, although linear /or logarithmic combinations of the first and second probabilities or highest/lowest value among the first and second probabilities might be considered to estimate the third probability P3.

In a step S6, the third probability P3 might be displayed on a navigation system display, with being superimposed to the electrocardiographic mapping ECM or the 3D model IH, for being interpretated by the cardiologist, helping him to accurately determine the most recommended treatment and/or to appreciate whether a part of the isthmus Rol should be considered as an ablation target area. As a variant, the third probability P3 might be storer in a computer memory for a later interpretation.

In undepicted embodiments, the segmentation step S2 might be a segmentation of an arrhythmogenic zone, as myocardium scar in which the electrical circulation is sustained according to a rhythm different from the cardiac sinus rhythm; a segmentation of an hypertrophic segment of a wall of heart's chamber, as a segment of the left ventricle myocardium; or a segmentation of an apical aneurysm or a thin-walled segment of the most distal portion of the left ventricle myocardium. The corresponding morphological features, and the appropriate machine learning algorithm might be adapted in any appropriate manner without departing from the scope of the disclosure.

The methods disclosed herein may also be implemented by software programs executable by a computer system. Further, implementations may include distributed processing and parallel processing, especially for processing in parallel several or all data in the data sets.

The illustrations described herein are intended to provide a general understanding of the structure of various embodiments. These illustrations are not intended to serve as a complete description of all the elements and features of apparatus, processors and systems that utilizes the structures or methods described therein. Many other embodiments or combinations thereof may be apparent to those of ordinary skills in the art upon reviewing the disclosure by combining the disclosed embodiments. Other embodiments may be utilized and derived from the disclosure, such that structural and logical substitutions and changes may be made without departing from the scope of the disclosure.

Further, the disclosure and the illustrations are to be considered as illustrative rather than restrictive, and the appended claims are intended to cover all such modifications, enhancements, and other embodiments, which fall within the true spirit and scope of the description. Thus, the scope of the following claims is to be determined by the broadest permissible interpretation of the claims and their equivalents and shall not be restricted or limited by the foregoing description.

## Claims

1. A computer-implemented method for the estimation of a risk of heart rhythm disorder in a patient's heart, the method comprising:
a. step of receiving at least one mapping of points representing said tissue of said patient's heart, each point being labelled with at least a value of at least one parameter and/or a classification indicating a local characteristic of said tissue at or around said point position;
b. step of receiving a set of electrograms signals, said set of electrograms signals being associated to a spatial location of said patient's heart;
c. step of registering said spatial location associated to said set of electrograms signals with the mapping of points;
d. step of computing a risk value indicating whether said patient's heart presents a heart rhythm disorder at said spatial location, wherein said risk value is computed from one or more of said electrograms signals and from the value of the parameter and/or the classification associated to at least one point of the mapping of points located at or around said registered spatial location.

2. The method according to claim 1, wherein said parameter value associated to a point of said mapping of points indicates a local thickness of said tissue at or around said point position.

3. The method according to one of claims 1 or 2, wherein said classification associated to a point of said mapping of points indicates the presence of a predetermined type of tissue in said tissue at or around said point position.

4. The method according to one of claims 1 to 3, wherein said step of risk value computing comprises:
a. a sub-step of extracting at least one timewise feature and/or one morphological feature from said one or more of said electrograms signals; and
b. a sub-step of implementing at least one trained machine learning algorithm on said at least one extracted feature to estimate a first probability indicating whether said patient's heart presents a heart rhythm disorder at said registered spatial location, wherein said trained machine learning algorithm have been trained to estimate, from a value of said at least one predetermined timewise and/or morphological feature of one or more electrograms signals, a probability indicating whether said patient's heart presents a heart rhythm disorder, and wherein said risk value is computed from said first probability.

5. The method according to claim 4, wherein said timewise feature extracting sub-step comprises the estimation of a value of a length cycle from said one or more of said electrograms signals.

6. The method according to one of claims 4 to 5, wherein said morphological feature extracting sub-step comprises the estimation of a norm of said one or more of said electrograms signals.

7. The method according to one of claims 4 to 6, wherein said step of risk value computing comprises a sub-step of adjusting the first probability following the value of the parameter and/or the classification associated to at least one point of the mapping of points located at or around said registered spatial location.

8. The method according to one of claims 4 to 6, wherein said step of risk value computing comprises:
a. a sub-step of estimating, from the value of the parameter and/or the classification associated to at least one point of the mapping of points located at or around said registered spatial location, a second probability indicating whether said patient's heart presents a heart rhythm disorder at said spatial location,
b. a sub-step of computing a third probability indicating whether said patient's heart presents a heart rhythm disorder with combining said first and second probabilities.

9. The method according to claim 8, wherein the second probability estimation sub-step comprises:
a. a sub-step of extracting, from a plurality of points of the mapping of points defining a region of interest located at or around said registered spatial location, a value of at least one predetermined morphological feature of said region of interest;
b. a sub-step of implementing a second trained machine learning algorithm on said extracted value to estimate said second probability, wherein said second trained machine learning algorithm have been trained to estimate, from a value of said at least one morphological feature of a region of interest of a patient's heart, a probability indicating whether said patient's heart presents a heart rhythm disorder.

10. The method according to one of claims 1 to 9, **characterized in that** it comprises, further to the step of risk value computing, a step of displaying on a navigation system display device said mapping of point with a landmark at the registered spatial location, wherein a visual feature of said landmark depends on said computed risk value.

11. The method according to one of claims 1 to 10, **characterized in that** it comprises, further to the step of risk value computing, a step of determining, from said risk value and from said mapping of points, an ablation target area at or around said registered spatial location.

12. A computing device for the implementation of the method according to one of claims 1 to 11, comprising a memory arranged to receive at least one mapping of points and a set of electrograms signals and a computing unit arranged to implement at least the registration step and the risk value computing step from said set of electrograms signals and said mapping of points.
